# EUROPEAN PATENT SPECIFICATION

(11) **EP 3 986 565 B1**
(45) Date of publication and mention of the grant of the patent: **30.08.2023**
(21) Application number: 20734486.2
(22) Date of filing: 19.06.2020
(51) Int. Cl.: A61P 35/00, C07D 417/14, A61K 31/427

(54) **NEW EGFR INHIBITORS**
NEUE EGFR-INHIBITOREN
NOUVEAUX INHIBITEURS DE L'EGFR

(30) Priority: 21.06.2019 EP 19181762
(43) Date of publication of application: 27.04.2022
(73) Proprietor: F. Hoffmann-La Roche AG, 4070 Basel (CH)
(72) Inventor: JAESCHKE, Georg, 4070 Basel (CH); KUHN, Bernd, 4070 Basel (CH); NAGEL, Yvonne Alice, 4070 Basel (CH); RICCI, Antonio, 4070 Basel (CH); RUEHER, Daniel, 4070 Basel (CH); STEINER, Sandra, 4070 Basel (CH)
(74) Representative: Salud, Carlos E.
(86) International application number: PCT/EP2020/067053
(87) International publication number: WO 2020/254544

(56) References cited:
- WO-A1-2018/220149
- US-A1- 2018 290 975

## Description

The present invention provides compounds which are selective allosteric inhibitors of T790M/L858R, T790M/L858R/C797S, L858R, L858R/C797S containing EGFR mutants, their manufacture, pharmaceutical compositions containing them and their use as therapeutically active substances.

The present invention provides a novel compound of formula (I) 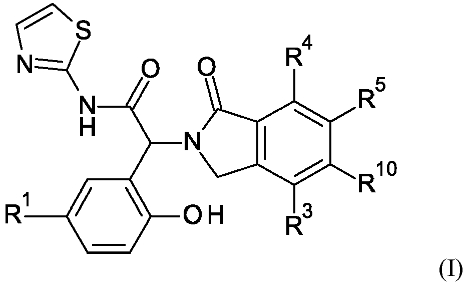 wherein
R¹ is selected from
   i) H,
   ii) C₁₋₆-alkyl, and
   iii) halogen;
R³ is halogen;
R⁴ is selected from
   i) H,
   ii) C₁₋₆-alkyl, and
   iii) halogen;
R⁵ is selected from
   i) phenyl substituted with R⁶ and optionally further substituted by R⁷ and R⁸,
   ii) pyridinyl substituted with R⁶ and optionally further substituted by R⁷ and R⁸, and
   iii) pyrimidinyl substituted with R⁶ and optionally further substituted by R⁷ and R8;
R⁶ is selected from
   i) piperazinyl optionally substituted by R⁹, R¹¹ and R¹²,
   ii) piperidinyl optionally substituted by R⁹, R¹¹ and R¹², and
   iii) morpholinyl optionally substituted by R⁹, R¹¹ and R¹²;
R⁷ and R⁸ are independently selected from
   i) C₁₋₆-alkyl,
   ii) C₁₋₆-alkoxy,
   iii) halo-C₁₋₆-alkyl,
   iv) halo-C₁₋₆-alkoxy,
   v) C₃₋₈-cycloalkyl,
   vi) C₃₋₈-cycloalkoxy,
   vii) halogen, and
   viii) hydroxy;
R⁹, R¹¹ and R¹² are independently selected from
   i) H,
   ii) halogen,
   iii) C₁₋₆-alkyl,
   iv) halo-C₁₋₆-alkyl, and
   v) C₃₋₈-cycloalkyl;
R¹⁰ is selected from
   i) H, and
   ii) C₁₋₆-alkyl,
   iii) C₃₋₈-cycloalkyl, and
   iv) halogen;
wherein halogen is at each instance independently selected from fluoro and chloro; or pharmaceutically acceptable salts. The following general disclosure also encompasses compounds, where halogen may be bromo or iodo. Such compounds are however not to be understood as forming part of the invention.

The HER family receptor tyrosine kinases are mediators of cell growth, differentiation and survival. The receptor family includes four distinct members, i.e. epidermal growth factor receptor (EGFR, ErbBl, or HER1) HER2 (ErbB2), HER3 (ErbB3) and HER4 (ErbB4). Upon ligand binding the receptors form homo and heterodimers and subsequent activation of the intrinsic tyrosine kinase activity leads to receptor auto-phosphorylation and the activation of downstream signaling molecules (Yarden, Y., Sliwkowski, MX. Untangling the ErbB signalling network. Nature Review Mol Cell Biol. 2001 Feb;2(2): 127-37). De-regulation of EGFR by overexpression or mutation has been implicated in many types of human cancer including colorectal, pancreatic, gliomas, head and neck and lung cancer, in particular non-small cell lung cancer (NSCLC) and several EGFR targeting agents have been developed over the years (Ciardiello, F., and Tortora, G. (2008). EGFR antagonists in cancer treatment. The New England journal of medicine 358, 1160-1174). Erlotinib (Tarceva^{®}), a reversible inhibitor of the EGFR tyrosine kinase was approved in numerous countries for the treatment of recurrent NSCLC.

An impressive single agent activity of EGFR tyrosine kinase inhibitors is observed in a subset of NSCLC patients whose tumors harbor somatic kinase domain mutations, whereas clinical benefit in wild-type EGFR patients is greatly diminished (Paez, J. et al. (2004). EGFR mutations in lung cancer: correlation with clinical response to gefitinib therapy. Science (New York, NY 304, 1497-1500). The most common somatic mutations of EGFR are exon 19 deletions with delta 746-750 the most prevalent mutation and the exon 21 amino acid substitutions with L858R the most frequent mutation (Sharma SV, Bell DW, Settleman J, Haber DA. Epidermal growth factor receptor mutations in lung cancer. Nat Rev Cancer. 2007 Mar;7(3): 169-81).

Treatment resistance arises frequently, often due to the secondary T790M mutation within the ATP site of the receptor. Some developed mutant-selective irreversible inhibitors are highly active against the T790M mutant, but their efficacy can be compromised by acquired mutation of C797S, that is the cysteine residue with which they form a key covalent bond (Thress, K. S. et al. Acquired EGFR C797S mutation mediates resistance to AZD9291 in non-small cell lung cancer harboring EGFR T790M. Nat. Med. 21, 560-562 (2015)). C797S mutation was further reported by Wang to be a major mechanism for resistance to T790M-targeting EGFR inhibitors (Wang et al. EGFR C797S mutation mediates resistance to third-generation inhibitors in T790M-positive non-small cell lung cancer, J Hematol Oncol. 2016; 9: 59). Additional mutations that cause resistance to Osimertinib are described by Yang, for example L718Q.( Yang et al, Investigating Novel Resistance Mechanisms to Third-Generation EGFR Tyrosine Kinase Inhibitor Osimertinib in Non-Small Cell Lung Cancer Patients, Clinical Cancer Research, DOI: 10.1158/1078-0432.CCR-17-2310) Lu et al.( Targeting EGFRL858R/T790M and EGFRL858R/T790M/C797S resistance mutations in NSCLC: Current developments in medicinal chemistry, Med Res Rev 2018; 1-32) report in a review article on Targeting EGFR^{L858R/T790M} and EGFR^{L858R/T790M/C797S} resistance mutations in NSCLC treatment.

As most available EGFR tyrosine kinase inhibitors target the ATP-site of the kinase, there is a need for new therapeutic agents that work differently, for example through targeting drug-resistant EGFR mutants.

Recent studies suggest that purposefully targeting allosteric sites might lead to mutant-selective inhibitors (Jia et al. Overcoming EGFR(T790M) and EGFR(C797S) resistance with mutant-selective allosteric inhibitoRS, June 2016, Nature 534, 129-132)

There is just a need in the generation of selective molecules that specifically inhibit T790M/L858R, T790M/L858R/C797S, L858R, L858R/C797S containing EGFR mutants useful for the therapeutic and/or prophylactic treatment of cancer, in particular T790M and C797S containing EGFR mutants. US2018290975 and WO2018220149 describe certain heteroaromatic compounds which allosterically modulate EGFR mutants.

The term "C₁₋₆-alkoxy" denotes a group of the formula -O-R', wherein R' is an C₁₋₆-alkyl group, particularly C₁₋₃-alkyl. Examples of C₁₋₆-alkoxy groups include methoxy, ethoxy, n-propoxy, isopropoxy, n-butoxy, isobutoxy and tert-butoxy. Particular examples are methoxy, ethoxy and isopropoxy. More particular example is methoxy.

The term "C₁₋₆-alkyl" denotes a monovalent linear or branched saturated hydrocarbon group of 1 to 6 carbon atoms, particularly 1 to 3 carbon atoms. Examples of C₁₋₆-alkylinclude methyl, ethyl, n-propyl, isopropyl, n-butyl, iso-butyl, sec-butyl, tert-butyl and pentyl. Particular C₁₋₆-alkylgroups are methyl, ethyl and isopropyl. More particular example is ethyl.

The term "C₃-₈-cycloalkoxy" denotes a group of the formula -O-R', wherein R' is a C₃₋₈-cycloalkyl group. Examples of cycloalkoxy group include cyclopropoxy, cyclobutoxy, cyclopentyloxy, cyclohexyloxy, cycloheptyloxy and cyclooctyloxy. Particular example is cyclopropoxy.

The term "C₃₋₈-cycloalkyl" denotes a monovalent saturated monocyclic or bicyclic hydrocarbon group of 3 to 8 ring carbon atoms. Bicyclic means a ring system consisting of two saturated carbocycles having one or two carbon atoms in common. Examples of monocyclic C₃₋₈-cycloalkyl are cyclopropyl, cyclobutanyl, cyclopentyl, cyclohexyl or cycloheptyl. Example of bicyclic C₃₋₈-cycloalkyl is spiro[3.3]heptanyl. Particular monocyclic C₃₋₈-cycloalkyl groups are cyclopropyl, cyclobutanyl. More particular monocyclic C₃₋₈-cycloalkyl groups include cyclopropyl.

The term "halo-C₁₋₆-alkoxy" denotes an C₁₋₆-alkoxy group wherein at least one of the hydrogen atoms of the C₁₋₆-alkoxy group has been replaced by same or different halogen atoms. The term "perhalo-C₁₋₆-alkoxy" denotes an C₁₋₆-alkoxy group where all hydrogen atoms of the C₁₋₆-alkoxy group have been replaced by the same or different halogen atoms. Examples of halo-C₁₋₆-alkoxy include fluoromethoxy, difluoromethoxy, trifluoromethoxy, fluoroethoxy, difluoroethoxy, trifluoroethoxy, trifluoromethylethoxy, trifluorodimethylethoxy and pentafluoroethoxy. Particular halo-C₁₋₆-alkoxy groups include fluoromethoxy, rifluoroethoxy, difluoromethoxy, difluoroethoxy, trifluoromethoxy, trifluoromethylethoxy and trifluorodimethylethoxy. More particular examples are fluoromethoxy, difluoromethoxy and trifluoromethoxy.

The term "halo-C₁₋₆-alkyl" denotes an C₁₋₆-alkylgroup wherein at least one of the hydrogen atoms of the C₁₋₆-alkylgroup has been replaced by the same or different halogen atoms. The term "perhalo-C₁₋₆-alkyl-C₁₋₆-alkyl" denotes an-C₁₋₆-alkyl-C₁₋₆-alkyl group where all hydrogen atoms of the alkyl group have been replaced by the same or different halogen atoms. Examples of halo-C₁₋₆-alkyl include fluoromethyl, difluoromethyl, trifluoromethyl, trifluoroethyl, trifluoromethylethyl and pentafluoroethyl. Particular halo-C₁₋₆-alkyl groups include fluoromethyl, difluoromethyl, trifluoromethyl, fluoroethyl, trifluoroethyl and difluoroethyl. More particular halo-C₁₋₆-alkyl groups include fluoromethyl.

The term "halogen" and "halo" are used interchangeably herein and denote fluoro, chloro, bromo or iodo. In the compounds according to the invention halogen is selected from fluoro and chloro.

The term "hydroxy" denotes a -OH group.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are the hydrochloride salts, methanesulfonic acid salts and citric acid salts.

"Pharmaceutically acceptable esters" means that compounds of general formula (I) may be derivatised at functional groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters. Additionally, any physiologically acceptable equivalents of the compounds of general formula (I), similar to the metabolically labile esters, which are capable of producing the parent compounds of general formula (I) in vivo, are within the scope of this invention.

The term "protecting group" (PG) denotes a group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. Particular protecting groups are the tert-butoxycarbonyl (Boc), benzyloxycarbonyl (Cbz), fluorenylmethoxycarbonyl (Fmoc) and benzyl (Bn) groups. Further particular protecting groups are the tert-butoxycarbonyl (Boc) and the fluorenylmethoxycarbonyl (Fmoc) groups. More particular protecting group is the tert-butoxycarbonyl (Boc) group.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention is a compound according to formula (I) as described herein and pharmaceutically acceptable salts or esters thereof, in particular compounds according to formula (I) as described herein and pharmaceutically acceptable salts thereof, more particularly compounds according to formula (I) as described herein.

A particular embodiment of the present invention provides a compound according to formula (I) as described herein, wherein
R¹ is halogen;
R³ is halogen;
R⁴ is selected from
   i) H, and
   ii) halogen;
R⁵ is phenyl substituted with R⁶,
R⁶ is piperidinyl optionally substituted by R⁹;
R⁹ is selected from
   i) C₁₋₆-alkyl, and
   ii) halo-C₁₋₆-alkyl,
or pharmaceutically acceptable salts.

A particular embodiment of the present invention provides a compound according to formula (I) as described herein, wherein R¹ is halogen.

A particular embodiment of the present invention provides a compound according to formula (I) as described herein, wherein R³ is halogen.

A particular embodiment of the present invention provides a compound according to formula (I) as described herein, wherein R⁵ is phenyl substituted with R⁶.

A more particular embodiment of the present invention provides a compound according to formula (I) as described herein, wherein R⁵ is phenyl substituted with R⁶.

A more particular embodiment of the present invention provides a compound according to formula (I) as described herein, wherein R⁶ is piperidinyl optionally substituted by R⁹.

A particular embodiment of the present invention provides a compound according to formula (I) as described herein, wherein R⁹ is selected from
i) C₁₋₆-alkyl, and
ii) halo-C₁₋₆-alkyl.

A particular embodiment of the present invention provides a compound as described herein, further defined as a compound of formula (II) or pharmaceutically acceptable salts.

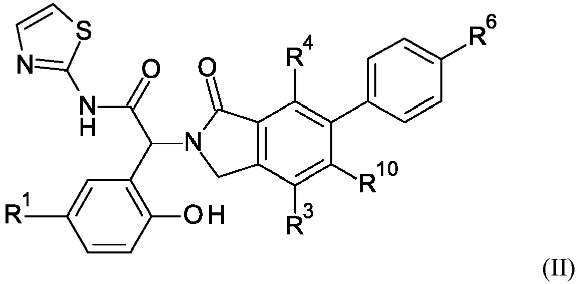

A more particular embodiment of the present invention provides a compound as described herein, further defined as a compound of formula (III) or pharmaceutically acceptable salts.

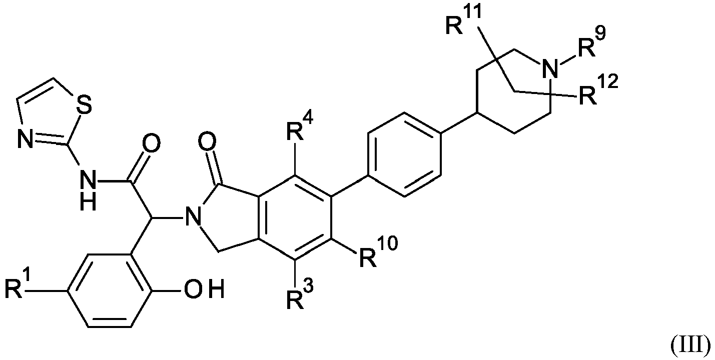

A furthermore particular embodiment of the present invention provides a compound as described herein, further defined as a compound of formula (IV) or pharmaceutically acceptable salts.

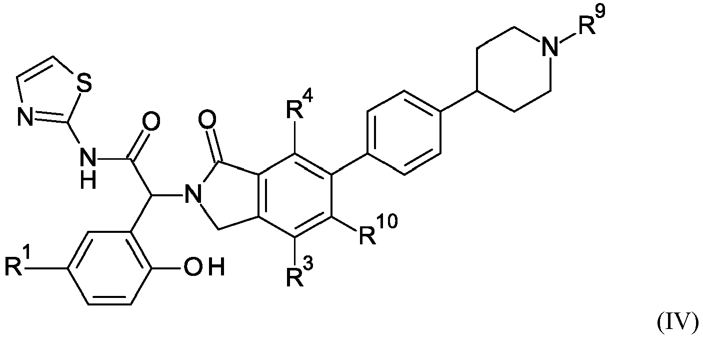

A furthermore particular embodiment of the present invention provides a compound according to formula (III) as described herein, wherein
R¹ is halogen;
R³ is halogen;
R⁴ is selected from
   i) H, and
   ii) halogen;
R⁹ is selected from
   i) C₁₋₆-alkyl, and
   ii) halo-C₁₋₆-alkyl,
or pharmaceutically acceptable salts.

A furthermore particular embodiment of the present invention provides a compound according to formula (IV) as described herein, wherein
R¹ is halogen;
R³ is halogen;
R⁴ is selected from
   iii) H, and
   iv) halogen;
R⁹ is selected from
   v) C₁₋₆-alkyl, and
   vi) halo-C₁₋₆-alkyl,
or pharmaceutically acceptable salts.

Particular examples of a compound of formula (I) as described herein are selected from
2-[6-[4-(1-ethyl-4-piperidyl)phenyl]-4-fluoro-1-oxo-isoindolin-2-yl]-2-(5-fluoro-2-hydroxyphenyl)-N-thiazol-2-yl-acetamide;
2-[7-chloro-6-[4-(1-ethyl-4-piperidyl)phenyl]-4-fluoro-1-oxo-isoindolin-2-yl]-2-(5-fluoro-2-hydroxy-phenyl)-N-thiazol-2-yl-acetamide;
2-[4-fluoro-6-[4-[1-(2-fluoroethyl)-4-piperidyl]phenyl]-1-oxo-isoindolin-2-yl]-2-(5-fluoro-2-hydroxy-phenyl)-N-thiazol-2-yl-acetamide;
or pharmaceutically acceptable salts thereof.

Processes for the manufacture of a compound of formula (I) as described herein are also an object of the invention.

The preparation of compounds of formula (I) of the present invention may be carried out in sequential or convergent synthetic routes. Syntheses of the invention are shown in the following general scheme. The skills required for carrying out the reactions and purifications of the resulting products are known to those skilled in the art. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The preparation of compounds of formula (I) of the present invention may be carried out in sequential or convergent synthetic routes. Syntheses of the compounds of the invention are shown in the following scheme 1 and in the description of specific examples. The skills required for carrying out the reactions and purifications of the resulting products are known to those skilled in the art. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

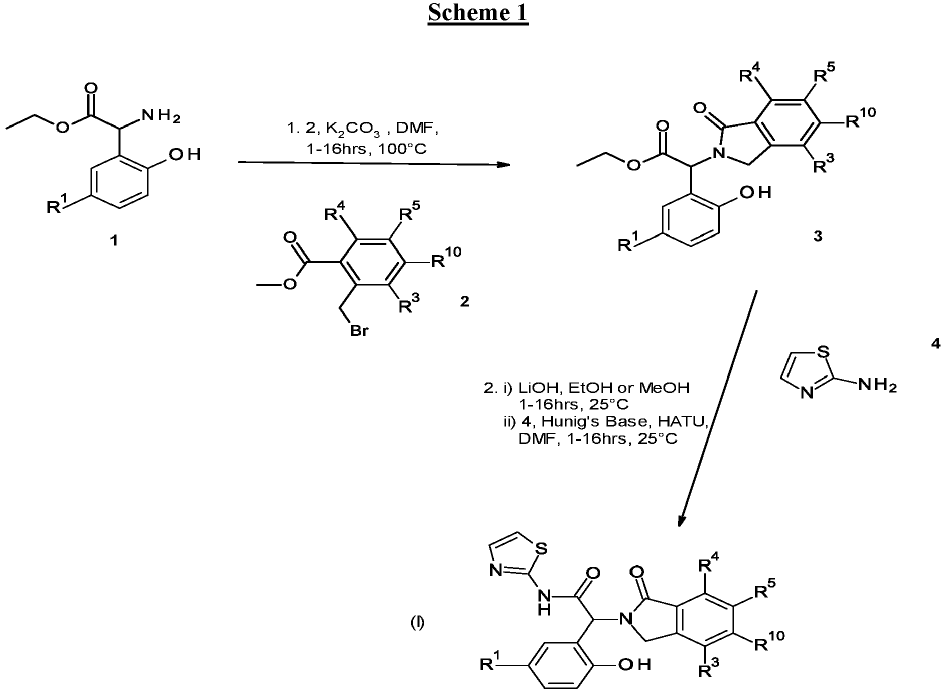

An isoindoline based compound of general formula I can be obtained for example by ring cyclization of a previously prepared aminoester 1 with an appropiately substituted methyl 2-(bromomethyl)benzoate of formula 2 to yield the desired isoindoline ester **3.** Saponification and amide coupling with an appropriately substituted amine of formula **4** with a coupling agent such as HATU yields the desired isoindoline compound of general formula **I** (scheme 1).

Generally speaking, the sequence of steps used to synthesize the compounds of formula **I,** and further functionalization can also be modified in certain cases.

The corresponding pharmaceutically acceptable salts with acids can be obtained by standard methods known to the person skilled in the art, e.g. by dissolving the compound of formula **I** in a suitable solvent such as e.g. dioxane or tetrahydrofuran and adding an appropriate amount of the corresponding acid. The products can usually be isolated by filtration or by chromatography. The conversion of a compound of formula **I** into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilisation. Particular salts are hydrochloride, formate and trifluoroacetate.

Insofar as their preparation is not described in the examples, the compounds of formula **I** as well as all intermediate products can be prepared according to analogous methods or according to the methods set forth herein. Starting materials are commercially available, known in the art or can be prepared by methods known in the art or in analogy thereto.

It will be appreciated that the compounds of general formula I in this invention may be derivatised at functional groups to provide derivatives which are capable of conversion back to the parent compound *in vivo.*

A certain embodiment of the invention relates to the compound of formula I as described herein, or a pharmaceutically acceptable salt thereof, for use as therapeutically active substance.

A certain embodiment of the invention relates to the compound of formula I as described herein, or a pharmaceutically acceptable salt thereof, for the use in the therapeutic and/or prophylactic treatment of cancer, in particular non-small-cell lung cancer.

A certain embodiment of the invention relates to the compound of formula I as described herein, or a pharmaceutically acceptable salt thereof, for the use in the therapeutic and/or prophylactic treatment of non-small-cell lung cancer.

A certain embodiment of the invention relates to the compound of formula I as described herein, or a pharmaceutically acceptable salt thereof, for the manufacture of a medicament for the therapeutic and/or prophylactic treatment of cancer, in particular non-small-cell lung cancer.

A certain embodiment of the invention relates to a pharmaceutical composition comprising the compound of formula I as described herein, or a pharmaceutically acceptable salt thereof, and a pharmaceutically acceptable auxiliary substance.

A certain embodiment of the invention relates to the compound of formula I as described herein, or a pharmaceutically acceptable salt thereof, for the use as a medicament in therapeutic and/or prophylactic treatment of a patient with EGFR activating mutations suffering from cancer, in particular non-small-cell lung cancer, comprising determining the EGFR activating mutations status in said patient and then administering the compound of formula I as described herein, or a pharmaceutically acceptable salt thereof, to said patient.

A certain embodiment of the invention relates to the compound of formula I as described herein, or a pharmaceutically acceptable salt thereof, for the use as a medicament in therapeutic and/or prophylactic treatment of a patient with EGFR mutations T790M/L858R, T790M/L858R/C797S, L858R and/or L858R/C797S suffering from cancer, in particular non-small-cell lung cancer, comprising determining the EGFR activating mutations status in said patient and then administering the compound of formula I as described herein, or a pharmaceutically acceptable salt thereof, to said patient.

A certain embodiment of the invention relates to the compound of formula I as described herein, or a pharmaceutically acceptable salt thereof, for the use as a medicament in therapeutic and/or prophylactic treatment of a patient with EGFR activating mutations as determined with a cobas^{®} EGFR Mutation Test v2 suffering from cancer, in particular non-small-cell lung cancer, comprising determining the EGFR activating mutations status in said patient and then administering the compound of formula I as described herein, or a pharmaceutically acceptable salt thereof, to said patient.

Furthermore, the invention includes all substituents in its corresponding deuterated form, wherever applicable, of the compounds of formula I.

Furthermore, the invention includes all optical isomers, i.e. diastereoisomers, diastereomeric mixtures, racemic mixtures, all their corresponding enantiomers and/or tautomers as well as their solvates, wherever applicable, of the compounds of formula I.

The compounds of formula I may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

Also an embodiment of the present invention are compounds of formula (I) as described herein, when manufactured according to any one of the described processes.

### Assay procedures

### HTRF Phospho EGFR TMLRCS assay (cellular)

### Cell line and media

BaF3-TMLRCS cell line were obtained from Crownbio (San Diego, CA, USA). Cells were maintained at 37°C, 5% CO₂ in RPMI ATCC (Gibco 31870) + 2mM Glutamine + 0.5µg/ml Puromycin supplemented with 10% fetal bovine serum (FBS) (Gibco).

### Protocol

Cells are transferred as above to Greiner Bio-One, Nr. 784-08 micro-titerplate at 20000 cells/well in 12.5 µl of growth medium/well after the plates had been pre-filled with 12.5 nl of DMSO solutions of the to be tested compounds (in dose response) or DMSO only. After spinning the plates at 300 x g for 30 seconds the cells were incubated for 4 hours at 37C, 5% CO2, 95% humidity. The cells were lysed by adding to the compound mix 4 µl/well of the supplemented lysis buffer (Cis-bio, Phospho-EGFR HTRF kit, 64EG1PEH), followed by incubation for 30 min at room temperature with shaking (400 rpm). The plates were then frozen and stored overnight at - 80C. On the next day and after thawing the plates, 4 µl of a mixture of anti-Phospho-EGFR Cryptate and of anti-Phospho-EGFR-d2 antibody solutions prepared in the supplied detection buffer was added to each well. The lidded plates were then incubated for 4 h at room temperature before reading the fluorescence emission at 616 and 665 nm using an Envision reader (Perkin Elmer). Data was analyzed in similar fashion as above using the normalized ratio of the 665 to 616 signals multiplied by 10000.

The results are shown in Table 1.

**Table 1: BaF3 cellular HTRF Phospho EGFR TMLRCS assay data**

| **Exam.** | **Structure** | **IC₅₀** (BaF3 TMLRCS) |
|---|---|---|
| 1 | 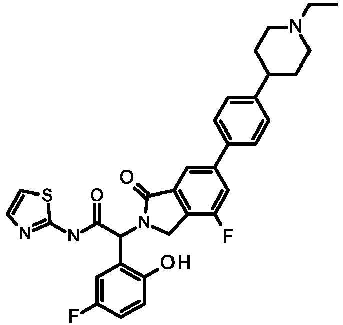 | 12nM |
| 2 | 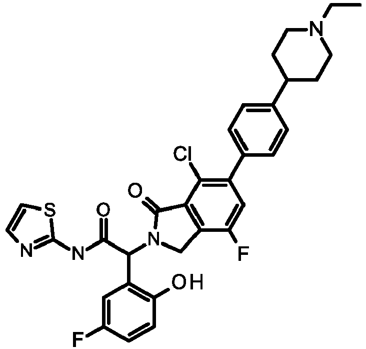 | 11nM |
| 3 | 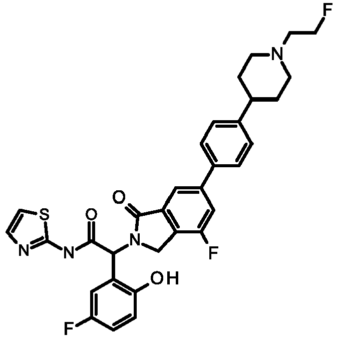 | 9nM |

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compounds of formula (I) and their pharmaceutically acceptable salts can be processed with pharmaceutically inert, inorganic or organic adjuvants for the production of tablets, coated tablets, dragees, hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Pharmaceutical Compositions

The compounds of formula I and the pharmaceutically acceptable salts can be used as therapeutically active substances, e.g. in the form of pharmaceutical preparations. The pharmaceutical preparations can be administered orally, e.g. in the form of tablets, coated tablets, dragees, hard and soft gelatin capsules, solutions, emulsions or suspensions. The administration can, however, also be effected rectally, e.g. in the form of suppositories, or parenterally, e.g. in the form of injection solutions.

The compounds of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula I. Examples of compositions according to the invention are:

### Example A

Tablets of the following composition are manufactured in the usual manner:

**Table 2: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 3: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 4: possible soft gelatin capsule ingredient composition**

| ingredient | mg/capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 5: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 6: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 7: possible injection solution composition**

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 8: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Experimental Part

The following examples are provided for illustration of the invention. They should not be considered as limiting the scope of the invention, but merely as being representative thereof.

### Example 1

### (2RS)-2-[6-[4-(1-Ethyl-4-piperidyl)phenyl]-4-fluoro-1-oxo-isoindolin-2-yl]-2-(5-fluoro-2-hydroxy-phenyl)-N-thiazol-2-yl-acetamide

### Step 1: tert-Butyl N-[(1RS)-1-(5-fluoro-2-methoxy-phenyl)-2-oxo-2-(thiazol-2-ylamino)ethyl]carbamate

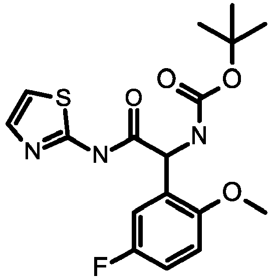

(2RS)-2-(Tert-butoxycarbonylamino)-2-(5-fluoro-2-methoxy-phenyl)acetic acid (2.2 g, 7.35 mmol) was dissolved in 22 ml ofDMF. Thiazol-2-amine (880 mg, 8.82 mmol, 1.2 equiv.), Hunig's base (3.85 ml, 22.1 mmol, 3 equiv.) and TBTU (2.6 g, 8.09 mmol, 1.1 equiv.) were added at room temperature. The mixture was stirred at room temperature for 18 hours. The reaction mixture was extracted with water and three times with ethyl acetate. The organic layers were extracted with water and 10% LiCl solution in water, dried over sodium sulfate and concentrated to dryness. The crude product was titurated in ether and dried to obtain the desired tert-butyl N-[(1RS)-1-(5-fluoro-2-methoxy-phenyl)-2-oxo-2-(thiazol-2-ylamino)ethyl]carbamate (2.4 g, 85 % yield) as a white solid, MS: m/e = 382.5 (M+H⁺).

### Step 2: (2RS)-2-Amino-2-(5-fluoro-2-methoxy-phenyl)-N-thiazol-2-yl-acetamide hydrochloride

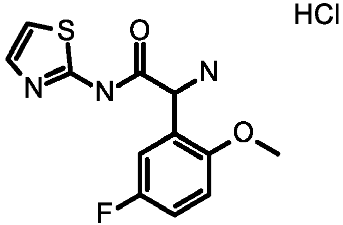

Tert-butyl N-[(1RS)-1-(5-fluoro-2-methoxy-phenyl)-2-oxo-2-(thiazol-2-ylamino)ethyl]carbamate *(Example 1, step 1)* (2.4 g, 6.27 mmol) was dissolved in 62 ml of methanol and HCl (4N in dioxane) (15.7 ml, 62.7 mmol, 10 equiv.) was added at room temperature. The mixture was stirred at 25 °C for 18 hours. The reaction mixture was concentrated under vacuum to give the desired (2RS)-2-amino-2-(5-fluoro-2-methoxy-phenyl)-N-thiazol-2-yl-acetamide hydrochloride (quant. yield) as a light green solid, MS: m/e = 282.4 (M+H⁺).

### Step 3: Methyl 5-iodo-2-(bromomethyl)-3-fluoro-benzoate

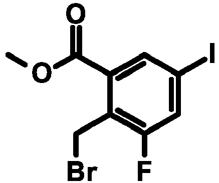

Methyl 5-iodo-3-fluoro-2-methylbenzoate (4.0 g, 13.0 mmol) was dissolved in 40 ml chlorobenzene and N-bromosuccinimide (3.45 g, 19.47 mmol, 1.5 equiv.) and AIBN (590 mg, 3.89 mmol, 0.3 equiv.) were added at room temperature. The mixture was stirred at 90°C for 12 hours. The reaction mixture was cooled, extracted with water and two times with ethyl acetate. The organic layers were dried over sodium sulfate and concentrated to dryness. The crude product was purified by flash chromatography on a silica gel column eluting with an ethyl acetate:heptane 0:100 to 50:50 gradient to obtain the desired methyl 5-iodo-2-(bromomethyl)-3-fluoro-benzoate.

### Step 4: (2RS)-2-(4-Fluoro-6-iodo-1-oxo-isoindolin-2-yl)-2-(5-fluoro-2-methoxy-phenyl)-N-thiazol-2-yl-acetamide

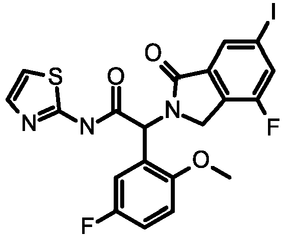

(2RS)-2-Amino-2-(5-fluoro-2-methoxy-phenyl)-N-thiazol-2-yl-acetamide hydrochloride *(Example 1, step 2)* (400 mg, 1.27 mmol, 1 equiv.) was dissolved in 2 ml ofDMF. Methyl 5-iodo-2-(bromomethyl)-3-fluoro-benzoate *(Example 1, step 3)* (474 mg, 1.27 mmol, 1 equiv.) and N,N-diisopropylethylamine (0.9 ml, 5.09 mmol, 4 equiv.) were added at room temperature. The mixture was stirred at room temperature for 90 minutes and at 70°C for an other 90 minutes. The reaction mixture was extracted with water and two times with ethyl acetate. The organic layers were extracted with brine, dried over sodium sulfate and concentrated to dryness. The crude product was purified by flash chromatography on a silica gel column eluting with a dichloromethane:methanol 100:0 to 90: 10 gradient to obtain the desired (2RS)-2-(4-fluoro-6-iodo-1-oxo-isoindolin-2-yl)-2-(5-fluoro-2-methoxy-phenyl)-N-thiazol-2-yl-acetamide (300 mg, 41 % yield) as a white solid, MS: m/e = 542.0 (M+H⁺).

### Step 5: 4-(4-Bromophenyl)-1-ethyl-piperidine

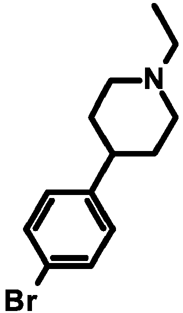

4-(4-Bromophenyl)piperidine (5 g, 20.8 mmol) was dissolved in 25 ml ofDMF. Ethyl iodide (1.85 ml, 22.9 mmol, 1.1 equiv.) and Hunig's base (7.3 ml, 41.6 mmol, 2 equiv.) were added at room temperature. The reaction mixture was stirred at room temperature for 2 hours. The reaction mixture was cooled to room temperature and then extracted with ethyl acetate and water. The aqueous layer was back-extracted with ethyl acetate. The organic layers were washed with brine. The organic layers were combined, dried over sodium sulfate, filtered and concentrated to dryness. The crude product was purified by flash chromatography on a silica gel column eluting with a dichloromethane:methanol 100:0 to 80:20 gradient to obtain the desired 4-(4-bromophenyl)-1-ethyl-piperidine (4.6 g, 78 % yield) as a yellow liquid, MS: m/e = 268.1/270.1 (M+H⁺).

### Step 6: 1-Ethyl-4-[4-(4,4,5,5-tetramethyl-1,3,2-dioxaborolan-2-yl)phenyl]piperidine

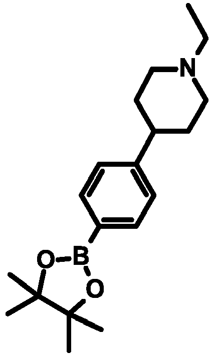

4-(4-Bromophenyl)-1-ethyl-piperidine *(Example 1, step 5)* (4.6 g, 15.4 mmol, 1.0 equiv.) and bis(pinacolato)diboron (4.31 g, 17 mmol, 1.1 equiv.) were dissolved in 40 ml of dioxane. Potassium acetate (4.54 g, 46.3 mmol, 3.0 equiv.) and dichloro 1,1'-bis(diphenylphosphino)ferrocene palladium(II) dichloromethane adduct (630 mg, 0.77 mmol, 0.05 equiv.) were added and the reaction mixture was stirred at 90°C for 20 hours. The reaction mixture was cooled to room temperature and concentrated to dryness. The crude product was purified by flash chromatography on a silica gel column eluting with a dichloromethane:methanol 100:0 to 80:20 gradient to obtain the desired product (quant. yield) as a dark brown oil, MS: m/e = 316.2 (M+H⁺).

### Step 7: (2RS)-2-[6-4-(1-Ethyl-4-piperidyl)phenyl]-4-fluoro-1-oxo-isoindolin-2-yl]-2-(5-fluoro-2-methoxy-phenyl)-N-thiazol-2-yl-acetamide

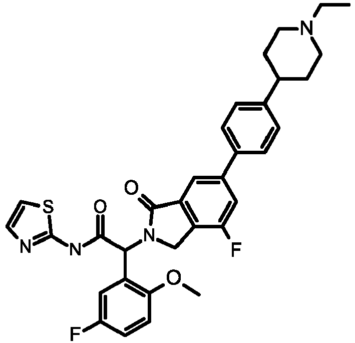

(2RS)-2-(4-Fluoro-6-iodo-1-oxo-isoindolin-2-yl)-2-(5-fluoro-2-methoxy-phenyl)-N-thiazol-2-yl-acetamide *(Example 1, step 4)* (150 mg, 0.277 mmol) and 1-ethyl-4-[4-(4,4,5,5-tetramethyl-1,3,2-dioxaborolan-2-yl)phenyl]piperidine *(Example 1, step 6)* (114 mg, 0.36 mmol, 1.3 equiv.) were dissolved in 4 ml of THF. Cesium carbonate (270 mg, 0.83 mmol, 3 equiv.), PdC1₂(dppf)-CH₂C1₂ adduct (23 mg, 0.028 mmol, 0.1 equiv.) and 1 ml of water were added and the reaction mixture was stirred at 65 °C for 24 hours. The reaction mixture was cooled to room temperature and then extracted with ethyl acetate and water. The aqueous layer was backextracted with ethyl acetate. The organic layers were washed with water and brine. The organic layers were combined, dried over sodium sulfate, filtered and concentrated on isolute^{®} to dryness. The crude product was purified by flash chromatography on a silica gel column eluting with a dichloromethane:methanol 100:0 to 80:20 gradient. The desired (2RS)-2-[6-[4-(1-ethyl-4-piperidyl)phenyl]-4-fluoro-1-oxo-isoindolin-2-yl]-2-(5-fluoro-2-methoxy-phenyl)-N-thiazol-2-yl-acetamide (160 mg, 67 % yield, purity = 70 %) was obtained as a dark green foam, MS: m/e = 603.3 (M+H⁺).

### Step 8: (2RS)-2-[6-[4-(1-Ethyl-4-piperidyl)phenyl]-4-fluoro-1-oxo-isoindolin-2-yl]-2-(5-fluoro-2-hydroxy-phenyl)-N-thiazol-2-yl-acetamide

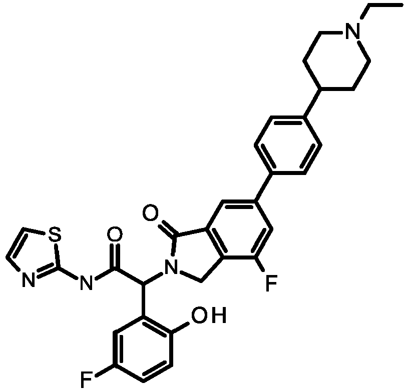

(2RS)-2-[6-[4-(1-Ethyl-4-piperidyl)phenyl]-4-fluoro-1-oxo-isoindolin-2-yl]-2-(5-fluoro-2-methoxy-phenyl)-N-thiazol-2-yl-acetamide *(Example 1, step 7)* (156 mg, 0.26 mmol) was dissolved in 5 ml of dichloromethane. BBr3 (1M in dichloromethane) (1.04 ml, 1.04 mmol, 4 equiv.) was added at room temperature. The mixture was stirred at 25°C for 3 hours. The reaction mixture was quenched by addition of saturated NaHCO3 solution in water. The reaction mixture was extracted with a mixture of dichloromethane:methanol 9:1. The aqueous layer was back-extracted three times with a mixture of dichloromethane:methanol 9: 1. The organic layers were combined, dried over sodium sulfate, filtered and concentrated to dryness. The crude product was purified by flash chromatography on a amino-silica gel column eluting with a dichloromethane:methanol 100:0 to 80:20 gradient to obtain the desired (2RS)-2-[6-[4-(1-ethyl-4-piperidyl)phenyl]-4-fluoro-1-oxo-isoindolin-2-yl]-2-(5-fluoro-2-hydroxy-phenyl)-N-thiazol-2-yl-acetamide (37 mg, 22 % yield) as a light yellow solid, MS: m/e = 589.2 (M+H⁺).

### Example 2

### (2RS)-2-[7-Chloro-6-[4-(1-ethyl-4-piperidyl)phenyl]-4-fluoro-1-oxo-isoindolin-2-yl]-2-(5-fluoro-2-hydroxy-phenyl)-N-thiazol-2-yl-acetamide

### Step 1: 2-Chloro-3-iodo-6-methyl-5-nitrobenzoic acid

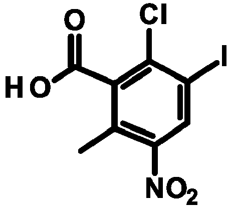

6-Chloro-2-methyl-3-nitrobenzoic acid (CAS 899821-27-3) (3.8 g, 15.9 mmol) was dissolved in 18 ml of sulfuric acid. 1,3-Diiodo-5,5-dimethylimidazolidine-2,4-dione (6.9 g, 18.2 mmol, 1.15 equiv.) was added at room temperature. The mixture was stirred at room temperature for 16 hours. The reaction mixture was poured onto water and the resulting precipitate filtered off. The solid was dried to obtain the desired 2-chloro-3-iodo-6-methyl-5-nitrobenzoic acid (5.37 g, quant. yield) as a brown foam, MS: m/e = 339.8/341.7 (M+H⁺).

### Step 2: Methyl 2-chloro-3-iodo-6-methyl-5-nitrobenzoate

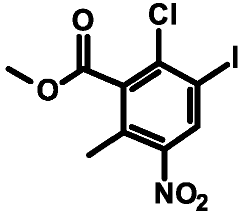

2-Chloro-3-iodo-6-methyl-5-nitrobenzoic acid *(Example 2, step 1)* (5.37 g, 14.2 mmol) was dissolved in 25 ml ofDMF. Potassium carbonate (3.9 g, 28.3 mmol, 2 equiv.) and iodomethane (2.1 g, 14.9 mmol, 1.05 equiv.) were added at room temperature. The mixture was stirred at room temperature for 2 hours. The reaction mixture was extracted with saturated sodiumbicarbonate solution and two times with ethyl acetate. The organic layers were extracted with water and 10% lithium chloride solution. The organic layers were combined, dried over sodium sulfate and concentrated to dryness. The crude product was purified by flash chromatography on a silica gel column eluting with a heptane:ethyl acetate 100:0 to 60:40 gradient to obtain the desired methyl 2-chloro-3-iodo-6-methyl-5-nitrobenzoate (2.89 g, 57 % yield) as a lighty yellow solid, MS: m/e = 353.9/355.9 (M+H⁺).

### Step 3: Methyl 3-amino-6-chloro-5-iodo-2-methylbenzoate

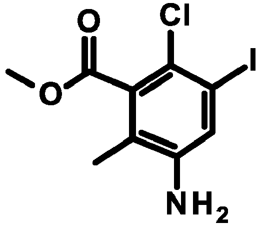

Methyl 2-chloro-3-iodo-6-methyl-5-nitrobenzoate *(Example 2, step 2)* (2.89 g, 8.13 mmol) was dissolved in 30 ml of methanol and 15 ml of water. Ammonium chloride (4.35 g, 81.3 mmol, 10 equiv.) and iron (2.72 g, 48.8 mmol, 6 equiv.) were added at room temperature. The mixture was stirred at 70°C for 16 hours. The reaction mixture was filtered and evaporated to dryness. The residue was extracted with saturated sodiumbicarbonate solution and two times with ethyl acetate. The organic layers were extracted with water and brine. The organic layers were combined, dried over sodium sulfate and concentrated to dryness. The crude product was purified by flash chromatography on a silica gel column eluting with a heptane:ethyl acetate 100:0 to 30:70 gradient to obtain the desired methyl 3-amino-6-chloro-5-iodo-2-methylbenzoate (1.9 g, 72 % yield) as an orange oil, MS: m/e = 324.9/326.9 (M+H⁺).

### Step 4: Methyl 2-chloro-5-fluoro-3-iodo-6-methylbenzoate (696 mg, 2.12 mmol, 49.1 % yield)

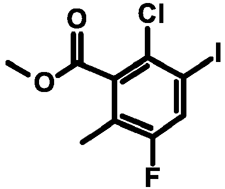

Methyl 3-amino-6-chloro-5-iodo-2-methylbenzoate *(Example 2, step 3)* (1.4 g, 4.3 mmol) was dissolved in 10 ml of dioxane. Nitrosonium tetrafluoroborate (0.55 g, 4.74 mmol, 1.1 equiv.) was added in portion and under ice cooling at room temperature. The mixture was stirred at room temperature for 30 minutes and at 110°C for 90 minutes. The reaction mixture was poured onto water and extracted twice with ethyl acetate. The organic layers were extracted with brine, dried over sodium sulfate and concentrated to dryness. The crude product was purified by flash chromatography on a silica gel column eluting with a heptane:ethyl acetate 100:0 to 75:25 gradient to obtain the desired methyl 2-chloro-5-fluoro-3-iodo-6-methylbenzoate (696 mg, 49 % yield) as a colorless oil, MS: m/e = 327.1/329.1 (M-H⁺).

### Step 5: Methyl 2-(bromomethyl)-6-chloro-3-fluoro-5-iodobenzoate

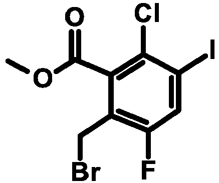

The title compound was obtained as a colorless solid, MS: m/e = 409.0 (M+H⁺), using chemistry similar to that described in Example 1, step 3 starting from methyl 2-chloro-5-fluoro-3-iodo-6-methylbenzoate *(Example 2, step 4).*

### Step 6: (2RS)-2-(7-Chloro-4-fluoro-6-iodo-1-oxo-isoindolin-2-yl)-2-(5-fluoro-2-methoxyphenyl)-N-thiazol-2-yl-acetamide

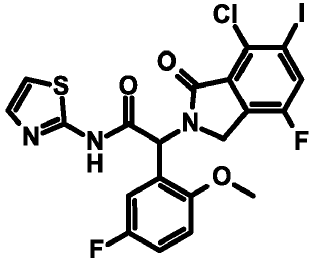

The title compound was obtained as a yellow solid, MS: m/e = 576.0 (M+H⁺), using chemistry similar to that described in Example 1, step 4 starting from (2RS)-2-amino-2-(5-fluoro-2-methoxy-phenyl)-N-thiazol-2-yl-acetamide hydrochloride *(Example 1, step 2)* and methyl 2-(bromomethyl)-6-chloro-3-fluoro-5-iodobenzoate *(Example 2, step 6).*

### Step 7: (2RS)-2-[7-Chloro-6-[4-(1-ethyl-4-piperidyl)phenyl]-4-fluoro-1-oxo-isoindolin-2-yl]-2-(5-fluoro-2-hydroxy-phenyl)-N-thiazol-2-yl-acetamide

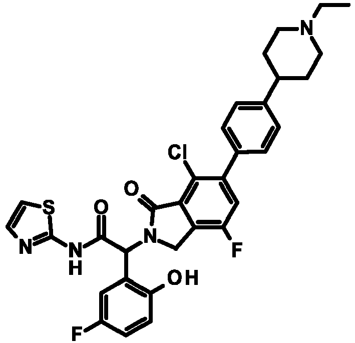

The title compound was obtained as a white solid, MS: m/e = 623.3 (M+H⁺), using chemistry similar to that described in Example 1, step 7 and step 8 starting from (2RS)-2-(7-Chloro-4-fluoro-6-iodo-1-oxo-isoindolin-2-yl)-2-(5-fluoro-2-methoxy-phenyl)-N-thiazol-2-yl-acetamide *(Example 2, step 6)* and 1-ethyl-4-[4-(4,4,5,5-tetramethyl-1,3,2-dioxaborolan-2-yl)phenyl]piperidine *(Example 1, step 6).*

### Example 3

### (2RS)-2-[4-Fluoro-6-[4-[1-(2-fluoroethyl)-4-piperidyl]phenyl]-1-oxo-isoindolin-2-yl]-2-(5-fluoro-2-hydroxy-phenyl)-N-thiazol-2-yl-acetamide

### Step 1: 1-(2-Fluoroethyl)-4-[4-(4,4,5,5-tetramethyl-1,3,2-dioxaborolan-2-yl)phenyl]piperidine

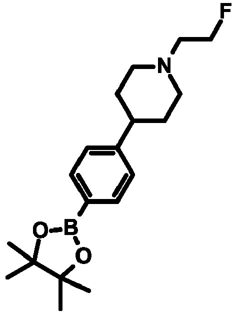

The title compound was obtained as a dark red solid, MS: m/e = 334.2 (M+H⁺), using chemistry similar to that described in Example 1, step 5 and step 6 starting from 4-(4-bromophenyl)piperidine and 2-fluoroethyl 4-methylbenzenesulfonate.

### Step 2: (2RS)-2-[4-Fluoro-6-[4-[1-(2-fluoroethyl)-4-piperidyl]phenyl]-1-oxo-isoindolin-2-yl]-2-(5-fluoro-2-hydroxy-phenyl)-N-thiazol-2-yl-acetamide

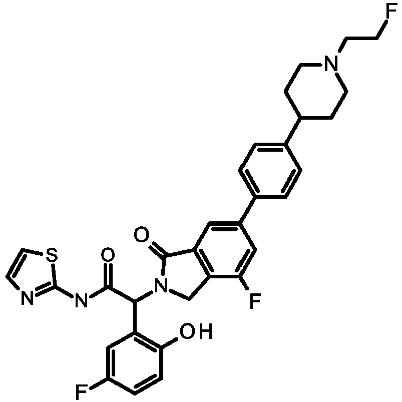

The title compound was obtained as a white solid, MS: m/e = 607.6 (M+H⁺), using chemistry similar to that described in Example 1, step 7 and step 8 starting from (2RS)-2-(4-fluoro-6-iodo-1-oxo-isoindolin-2-yl)-2-(5-fluoro-2-methoxy-phenyl)-N-thiazol-2-yl-acetamide *(Example 1, step 4)* and 1-(2-fluoroethyl)-4-[4-(4,4,5,5-tetramethyl-1,3,2-dioxaborolan-2-yl)phenyl]piperidine *(Example 3, step 1).*

## Claims

1. A compound of formula (I) 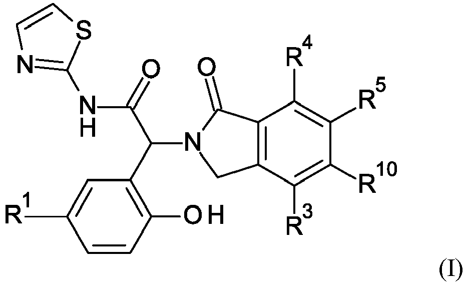 wherein
R¹ is selected from
i) H,
ii) C₁₋₆-alkyl, and
iii) halogen;
R³ is halogen;
R⁴ is selected from
i) H,
ii) C₁₋₆-alkyl, and
iii) halogen;
R⁵ is selected from
i) phenyl substituted with R⁶ and optionally further substituted by R⁷ and R⁸,
ii) pyridinyl substituted with R⁶ and optionally further substituted by R⁷ and R⁸, and
iii) pyrimidinyl substituted with R⁶ and optionally further substituted by R⁷ and R8;
R⁶ is selected from
i) piperazinyl optionally substituted by R⁹, R¹¹ and R¹²,
ii) piperidinyl optionally substituted by R⁹, R¹¹ and R¹², and
iii) morpholinyl optionally substituted by R⁹, R¹¹ and R¹²;
R⁷ and R⁸ are independently selected from
i) C₁₋₆-alkyl,
ii) C₁₋₆-alkoxy,
iii) halo-C₁₋₆-alkyl,
iv) halo-C₁₋₆-alkoxy,
v) C₃₋₈-cycloalkyl,
vi) C₃₋₈-cycloalkoxy,
vii) halogen, and
viii) hydroxy;
R⁹, R¹¹ and R¹² are independently selected from
i) H,
ii) halogen,
iii) C₁₋₆-alkyl,
iv) halo-C₁₋₆-alkyl,and
v) C₃₋₈-cycloalkyl;
R¹⁰ is selected from
i) H, and
ii) C₁₋₆-alkyl,
iii) C₃₋₈-cycloalkyl, and
iv) halogen;
wherein halogen is at each instance independently selected from fluoro and chloro; or pharmaceutically acceptable salts.

2. A compound according to claim 1, wherein R¹ is halogen selected from fluoro and chloro.

3. A compound according to any one of claims 1 to 2, wherein R⁵ is phenyl substituted with R6.

4. A compound according to any one of claims 1 to 3, wherein R⁶ is piperidinyl optionally substituted by R⁹.

5. A compound according to any one of claims 1 to 4, wherein R⁹ is selected from
i) C₁₋₆-alkyl, and
ii) halo-C₁₋₆-alkyl.

6. A compound of formula (I) according to claim 1, wherein
R¹ is halogen;
R³ is halogen;
R⁴ is selected from
i) H, and
ii) halogen;
R⁵ is phenyl substituted with R⁶,
R⁶ is piperidinyl optionally substituted by R⁹;
R⁹ is selected from
i) C₁₋₆-alkyl, and
ii) halo-C₁₋₆-alkyl;
R¹⁰ is halogen;
wherein halogen is at each instance independently selected from fluoro and chloro; or pharmaceutically acceptable salts.

7. A compound according to claim 1 further defined as a compound of formula (II), 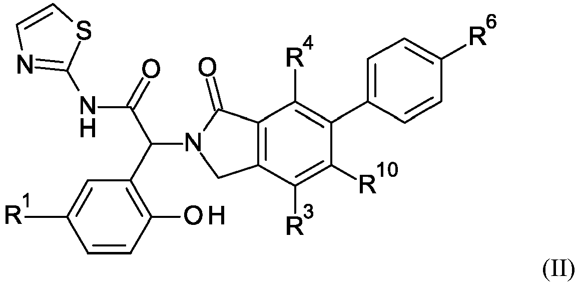 or pharmaceutically acceptable salts.

8. A compound according to any one of claims 1 or 7 further defined as a compound of formula (IV), 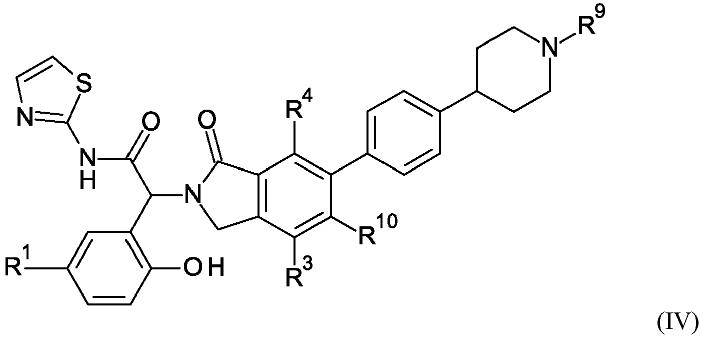 or pharmaceutically acceptable salts.

9. A compound according to claims 9, wherein
R¹ is halogen;
R³ is halogen;
R⁴ is selected from
i) H, and
ii) halogen;
R⁹ is selected from
i) C₁₋₆-alkyl, and
ii) halo-C₁₋₆-alkyl,
R¹⁰ is H;
wherein halogen is at each instance independently selected from fluoro and chloro;
or pharmaceutically acceptable salts.

10. A compound according to any one of claims 1 to 9, selected from
2-[6-[4-(1-ethyl-4-piperidyl)phenyl]-4-fluoro-1-oxo-isoindolin-2-yl]-2-(5-fluoro-2-hydroxyphenyl)-N-thiazol-2-yl-acetamide;
2-[7-chloro-6-[4-(1-ethyl-4-piperidyl)phenyl]-4-fluoro-1-oxo-isoindolin-2-yl]-2-(5-fluoro-2-hydroxy-phenyl)-N-thiazol-2-yl-acetamide;
2-[4-fluoro-6-[4-[1-(2-fluoroethyl)-4-piperidyl]phenyl]-1-oxo-isoindolin-2-yl]-2-(5-fluoro-2-hydroxy-phenyl)-N-thiazol-2-yl-acetamide;
or pharmaceutically acceptable salts.

11. A compound according to any one of claims 1 to 10 for use as therapeutically active substance.

12. A pharmaceutical composition comprising a compound according to any one of claims 1 to 10 and a therapeutically inert carrier.

13. A compound according to any one of claims 1 to 10 for use in the treatment or prophylaxis of cancer.

14. A compound according to any one of claims 1 to 10 for use in the treatment or prophylaxis of non-small cell lung cancer.

15. A compound according to any one of claims 1 to 10 for the use as a medicament in therapeutic and/or prophylactic treatment of a patient with EGFR activating mutations suffering from cancer, in particular non-small-cell lung cancer, comprising determining the EGFR activating mutations status in said patient and then administering a compound according to any one of claims 1 to 10 to said patient.

## Patentansprüche

1. Verbindung der Formel (I) 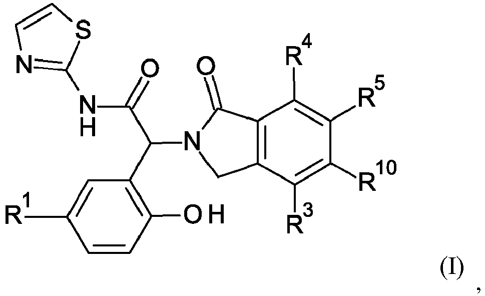 wobei
R¹ aus
i) H,
ii) C₁₋₆-Alkyl und
iii) Halogen
ausgewählt ist;
R³ Halogen ist;
R⁴ aus
i) H,
ii) C₁₋₆-Alkyl und
iii) Halogen
ausgewählt ist;
R⁵ aus
i) Phenyl, das mit R⁶ substituiert ist und gegebenenfalls ferner mit R⁷ und R⁸ substituiert ist;
ii) Pyridinyl, das mit R⁶ substituiert ist und gegebenenfalls ferner mit R⁷ und R⁸ substituiert ist; und
iii) Pyrimidinyl, das mit R⁶ substituiert ist und gegebenenfalls ferner mit R⁷ und R⁸ substituiert ist,
ausgewählt ist;
R⁶ aus
i) Piperazinyl, das gegebenenfalls mit R⁹, R¹¹ und R¹² substituiert ist;
ii) Piperidinyl, das gegebenenfalls mit R⁹, R¹¹ und R¹² substituiert ist; und
iii) Morpholinyl, das gegebenenfalls mit R⁹, R¹¹ und R¹² substituiert ist,
ausgewählt ist;
R⁷ und R⁸ unabhängig voneinander aus
i) C₁₋₆-Alkyl,
ii) C₁₋₆-Alkoxy,
iii) Halogen-C₁₋₆-alkyl,
iv) Halogen-C₁₋₆-alkoxy,
v) C₃₋₈-Cycloalkyl,
vi) C₃₋₈-Cycloalkoxy,
vii) Halogen und
viii) Hydroxy
ausgewählt sind;
R⁹, R¹¹ und R¹² unabhängig voneinander aus
i) H,
ii) Halogen,
iii) C₁₋₆-Alkyl,
iv) Halogen-C₁₋₆-alkyl und
v) C₃₋₈-Cycloalkyl
ausgewählt sind;
R¹⁰ aus
i) H und
ii) C₁₋₆-Alkyl,
iii) C₃₋₈-Cycloalkyl und
iv) Halogen
ausgewählt ist;
wobei Halogen bei jedem Auftreten unabhängig voneinander aus Fluor und Chlor ausgewählt ist;
oder pharmazeutisch unbedenkliche Salze.

2. Verbindung nach Anspruch 1, wobei R¹ Halogen ist, das aus Fluor und Chlor ausgewählt ist.

3. Verbindung nach einem der Ansprüche 1 bis 2, wobei R⁵ Phenyl ist, das mit R⁶ substituiert ist.

4. Verbindung nach einem der Ansprüche 1 bis 3, wobei R⁶ Piperidinyl ist, das gegebenenfalls mit R⁹ substituiert ist.

5. Verbindung nach einem der Ansprüche 1 bis 4, wobei R⁹ aus
i) C₁₋₆-Alkyl und
ii) Halogen-C₁₋₆-alkyl
ausgewählt ist.

6. Verbindung der Formel (I) nach Anspruch 1, wobei
R¹ Halogen ist;
R³ Halogen ist;
R⁴ aus
i) H und
ii) Halogen
ausgewählt ist;
R⁵ Phenyl ist, das mit R⁶ substituiert ist,
R⁶ Piperidinyl ist, das gegebenenfalls mit R⁹ substituiert ist;
R⁹ aus
i) C₁₋₆-Alkyl und
ii) Halogen-C₁₋₆-alkyl
ausgewählt ist;
R¹⁰ Halogen ist;
wobei Halogen bei jedem Auftreten unabhängig voneinander aus Fluor und Chlor ausgewählt ist;
oder pharmazeutisch unbedenkliche Salze.

7. Verbindung nach Anspruch 1, die ferner als eine Verbindung der Formel (II) definiert ist 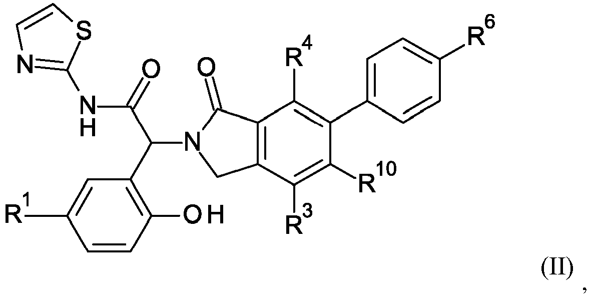 oder pharmazeutisch unbedenkliche Salze.

8. Verbindung nach einem der Ansprüche 1 oder 7, die ferner als eine Verbindung der Formel (IV) definiert ist 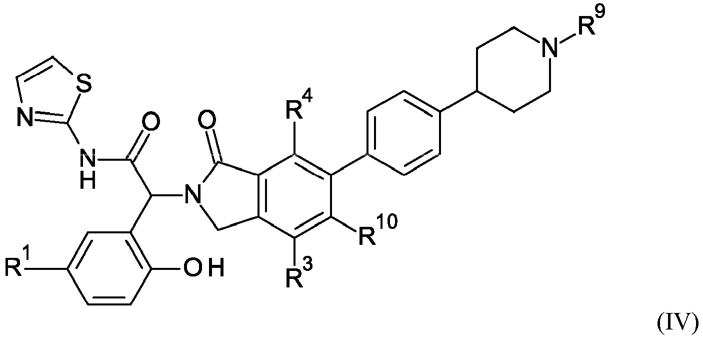 oder pharmazeutisch unbedenkliche Salze.

9. Verbindung nach den Ansprüchen 9, wobei
R¹ Halogen ist;
R³ Halogen ist;
R⁴ aus
i) H und
ii) Halogen
ausgewählt ist;
R⁹ aus
i) C₁₋₆-Alkyl und
ii) Halogen-C₁₋₆-alkyl
ausgewählt ist,
R¹⁰ H ist;
wobei Halogen bei jedem Auftreten unabhängig voneinander aus Fluor und Chlor ausgewählt ist;
oder pharmazeutisch unbedenkliche Salze.

10. Verbindung nach einem der Ansprüche 1 bis 9, ausgewählt aus
2-[6-[4-(1-Ethyl-4-piperidyl)phenyl]-4-fluor-1-oxo-isoindolin-2-yl]-2-(5-fluor-2-hydroxyphenyl)-N-thiazol-2-yl-acetamid;
2-[7-Chlor-6-[4-(1-ethyl-4-piperidyl)phenyl]-4-fluor-1-oxo-isoindolin-2-yl]-2-(5-fluor-2-hydroxyphenyl)-N-thiazol-2-yl-acetamid;
2-[4-Fluor-6-[4-[1-(2-fluorethyl)-4-piperidyl]phenyl]-1-oxo-isoindolin-2-yl]-2-(5-fluor-2-hydroxyphenyl)-N-thiazol-2-yl-acetamid;
oder pharmazeutisch unbedenklichen Salzen.

11. Verbindung nach einem der Ansprüche 1 bis 10 zur Verwendung als therapeutisch wirksame Substanz.

12. Pharmazeutische Zusammensetzung, umfassend eine Verbindung nach einem der Ansprüche 1 bis 10 und einen therapeutisch inerten Träger.

13. Verbindung nach einem der Ansprüche 1 bis 10 zur Verwendung bei der Behandlung oder Prophylaxe von Krebs.

14. Verbindung nach einem der Ansprüche 1 bis 10 zur Verwendung bei der Behandlung oder Prophylaxe von nichtkleinzelligem Lungenkrebs.

15. Verbindung nach einem der Ansprüche 1 bis 10 zur Verwendung als ein Medikament bei der therapeutischen und/oder prophylaktischen Behandlung eines Patienten mit EGFR-aktivierenden Mutationen, der an Krebs, insbesondere nichtkleinzelligem Lungenkrebs, leidet, umfassend das Bestimmen des Status der EGFR-aktivierenden Mutationen bei dem Patienten und dann das Verabreichen einer Verbindung nach einem der Ansprüche 1 bis 10 an den Patienten.

## Revendications

1. Composé de formule (I) 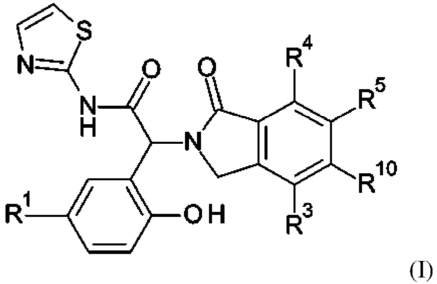 dans lequel
R¹ est choisi parmi
i) H,
ii) un alkyle en C₁₋₆, et
iii) un halogène ;
R³ représente un halogène ;
R⁴ est choisi parmi
i) H,
ii) un alkyle en C₁₋₆, et
iii) un halogène ;
R⁵ est choisi parmi
i) un phényle substitué par R⁶ et en outre éventuellement substitué par R⁷ et R⁸,
ii) un pyridinyle substitué par R⁶ et en outre éventuellement substitué par R⁷ et R⁸,et
iii) un pyrimidinyle substitué par R⁶ et en outre éventuellement substitué par R⁷ et R⁸;
R⁶ est choisi parmi
i) un pipérazinyle éventuellement substitué par R⁹, R¹¹ et R¹²,
ii) un pipéridinyle éventuellement substitué par R⁹, R¹¹ et R¹², et
iii) un morpholinyle éventuellement substitué par R⁹, R¹¹ et R¹² ;
R⁷ et R⁸ sont indépendamment choisis parmi
i) un alkyle en C₁₋₆,
ii) un alcoxy en C₁₋₆,
iii) un halogénoalkyle en C₁₋₆,
iv) un halogénoalcoxy en C₁₋₆,
v) un cycloalkyle en C₃₋₈,
vi) un cycloalcoxy en C₃₋₈,
vii) un halogène, et
viii) un hydroxy ;
R⁹, R¹¹ et R¹² sont indépendamment choisis parmi
i) H,
ii) un halogène,
iii) un alkyle en C₁₋₆,
iv) un halogénoalkyle en C₁₋₆, et
v) un cycloalkyle en C₃₋₈ ;
R¹⁰ est choisi parmi
i) H, et
ii) un alkyle en C₁₋₆,
iii) un cycloalkyle en C₃₋₈, et
iv) un halogène ;
dans lequel l'halogène est dans chaque cas indépendamment choisi parmi un fluor et un chlore ;
ou des sels pharmaceutiquement acceptables.

2. Composé selon la revendication 1, dans lequel R¹ représente un halogène choisi parmi un fluor et un chlore.

3. Composé selon l'une quelconque des revendications 1 à 2, dans lequel R⁵ représente un phényle substitué par R⁶.

4. Composé selon l'une quelconque des revendications 1 à 3, dans lequel R⁶ représente un pipéridinyle éventuellement substitué par R⁹.

5. Composé selon l'une quelconque des revendications 1 à 4, dans lequel R⁹ est choisi parmi
i) un alkyle en C₁₋₆, et
ii) un halogénoalkyle en C₁₋₆.

6. Composé de formule (I) selon la revendication 1, dans lequel
R¹ représente un halogène ;
R³ représente un halogène ;
R⁴ est choisi parmi
i) H, et
ii) un halogène ;
R⁵ représente un phényle substitué par R⁶,
R⁶ représente un pipéridinyle éventuellement substitué par R⁹ ;
R⁹ est choisi parmi
i) un alkyle en C₁₋₆, et
ii) un halogénoalkyle en C₁₋₆ ;
R¹⁰ représente un halogène ;
dans lequel l'halogène est dans chaque cas indépendamment choisi parmi un fluor et un chlore ;
ou des sels pharmaceutiquement acceptables.

7. Composé selon la revendication 1 défini en outre comme un composé de formule (II), 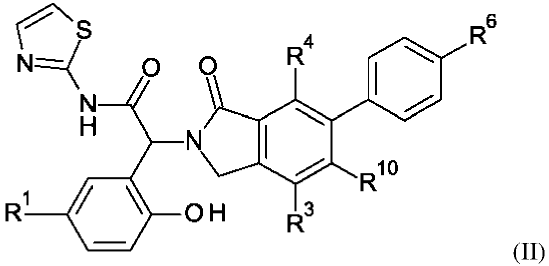 ou des sels pharmaceutiquement acceptables.

8. Composé selon l'une quelconque des revendications 1 à 7 défini en outre comme un composé de formule (IV), 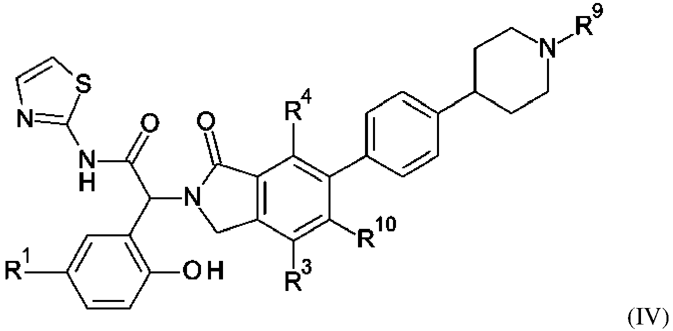 ou des sels pharmaceutiquement acceptables.

9. Composé selon les revendications 9, dans lequel
R¹ représente un halogène ;
R³ représente un halogène ;
R⁴ est choisi parmi
i) H, et
ii) un halogène ;
R⁹ est choisi parmi
i) un alkyle en C₁₋₆, et
ii) un halogénoalkyle en C₁₋₆,
R¹⁰ représente H ;
dans lequel l'halogène est dans chaque cas indépendamment choisi parmi un fluor et un chlore ; ou des sels pharmaceutiquement acceptables.

10. Composé selon l'une quelconque des revendications 1 à 9, choisi parmi
le 2-[6-[4-(1-éthyl-4-pipéridyl)phényl]-4-fluoro-1-oxo-isoindolin-2-yl]-2-(5-fluoro-2-hydroxy-phényl)-N-thiazol-2-yl-acétamide ;
le 2-[7-chloro-6-[4-(1-éthyl-4-pipéridyl)phényl]-4-fluoro-1 -oxo-isoindolin-2-yl] -2-(5-fluoro-2-hydroxy-phényl)-N-thiazol-2-yl-acétamide ;
le 2-[4-fluoro-6-[4-[1-(2-fluoroéthyl)-4-pipéridyl]phényl]-1-oxo-isoindolin-2-yl]-2-(5-fluoro-2-hydroxy-phényl)-N-thiazol-2-yl-acétamide ;
ou des sels pharmaceutiquement acceptables.

11. Composé selon l'une quelconque des revendications 1 à 10 pour une utilisation comme substance thérapeutiquement active.

12. Composition pharmaceutique comprenant un composé selon l'une quelconque des revendications 1 à 10 et un véhicule thérapeutiquement inerte.

13. Composé selon l'une quelconque des revendications 1 à 10 pour une utilisation dans le traitement ou la prophylaxie d'un cancer.

14. Composé selon l'une quelconque des revendications 1 à 10 pour une utilisation dans le traitement ou la prophylaxie d'un cancer du poumon non à petites cellules.

15. Composé selon l'une quelconque des revendications 1 à 10 pour une utilisation comme médicament dans le traitement thérapeutique et/ou prophylactique d'un patient avec des mutations activatrices de l'EGFR souffrant d'un cancer, en particulier d'un cancer du poumon non à petites cellules, comprenant la détermination de l'état des mutations activatrices de l'EGFR chez ledit patient et ensuite l'administration d'un composé selon l'une quelconque des revendications 1 à 10 audit patient.
